# EUROPEAN PATENT APPLICATION

(11) **EP 3 505 610 A1**
(43) Date of publication of application: **03.07.2019**
(21) Application number: 17843704.2
(22) Date of filing: 24.08.2017
(51) Int. Cl.: C12M 1/00, C12M 1/02, C12M 3/02, C12N 5/00

(54) **CELL CULTURE DEVICE AND CELL CULTURE METHOD**

(30) Priority: 26.08.2016 JP 2016166068
(71) Applicant: IHI Corporation, Tokyo 135-8710 (JP)
(72) Inventor: ISHII Kousuke, Tokyo 135-8710 (JP); YOSHIMURA Akihiko, Tokyo 135-8710 (JP); SAITO Makiko, Tokyo 135-8710 (JP); SHIDA Michinori, Tokyo 135-8710 (JP); NAKAGAWA Masayo, Tokyo 135-8710 (JP); SAKAI Shin-ichi, Tokyo 135-8710 (JP)
(74) Representative: Grünecker Patent- und Rechtsanwälte PartG mbB
(86) International application number: PCT/JP2017/030410
(87) International publication number: WO 2018/038228

(57) **Abstract**

A cell culture device includes: a culture tank of culturing a cell in a culture solution, the culture tank being in a shape in which a horizontal sectional area upwardly increases; a pump and a culture solution supply pipe, as a supply unit supplying the culture solution to the culture tank; and a control unit controlling the supply of the culture solution of the supply unit, in which the culture solution is intermittently supplied to the culture tank from the supply unit, according to the control of the control unit, and a circulation upward flow is formed in the culture solution, and thus, a plug flow of the culture solution is formed in a culture region in which the cell is retained in the culture tank.

## Description

### Technical Field

The present disclosure relates to a cell culture device and a cell culture method. This application is based upon and claims the benefit of priority from Japanese Patent Application No. 2016-166068, filed on Aug. 26, 2016 and claims the benefit of priority thereto; the entire contents of which are incorporated herein by reference.

### Background Art

A cell culture device in which a culture solution is supplied from a bottom portion of a culture tank, and an upward flow of the culture solution is formed to be balanced with a sedimentation rate of a cell, by using the culture tank of which a horizontal sectional surface upwardly increases, and thus, the cell is three-dimensionally cultured in the culture tank, has been known as a device culturing a cell such as an animal cell or a plant cell. In addition, a method of controlling the flow of the culture solution in the cell culture device, is described in Patent Literature 1.

### Citation List

### Patent Literature

Patent Literature 1: Japanese Unexamined Patent Publication No. 2015-142550

### Summary of Invention

### Technical Problem

However, in the cell culture device described above, in a case where the upward flow of the culture solution is controlled according to the growth of the cell from when the culture is started, it is necessary to greatly change a flow rate of the culture solution to be supplied to the cell culture device. In addition, a possibility that the flow of the culture solution in the culture tank is destabilized due to a change in the flow rate of the culture solution, is considered.

In the present disclosure, a cell culture device and a cell culture method, in which it is possible to control a supply amount of a culture solution according to the growth of a cell while stabilizing the flow of the culture solution in a culture tank, will be described.

### Solution to Problem

A cell culture device according to one aspect of the present disclosure, includes: a culture tank of culturing a cell in a culture solution, the culture tank being in a shape in which a horizontal sectional area upwardly increases; a supply unit supplying the culture solution to the culture tank; and a control unit controlling the supply of the culture solution of the supply unit, in which the culture solution is intermittently supplied to the culture tank from the supply unit, according to the control of the control unit, and a circulation upward flow is formed in the culture solution, and thus, a plug flow of the culture solution is formed in a culture region in which the cell is retained in the culture tank.

### Effects of Invention

According to the present disclosure, it is possible to provide a cell culture device in which it is possible to control a supply amount of a culture solution according to the growth of a cell while stabilizing the flow of the culture solution in a culture tank.

### Brief Description of Drawings

FIG. 1 is a diagram illustrating a schematic configuration of a cell culture device according to one embodiment of the present disclosure.
FIG. 2 is a diagram illustrating a movement of a culture solution in a culture tank.
FIG. 3 is a diagram illustrating intermittent supply of the culture solution.
FIG. 4 is a diagram illustrating a schematic configuration of a cell culture device according to a modification example.
FIG. 5 is a diagram illustrating a schematic configuration of the cell culture device according to the modification example.

### Description of Embodiments

In order to attain the object described above, a cell culture device according to one aspect of the present disclosure, includes: a culture tank of culturing a cell in a culture solution, the culture tank being in a shape in which a horizontal sectional area upwardly increases; a supply unit supplying the culture solution to the culture tank; and a control unit controlling the supply of the culture solution of the supply unit, in which the culture solution is intermittently supplied to the culture tank from the supply unit, according to the control of the control unit, and a circulation upward flow is formed in the culture solution, and thus, a plug flow of the culture solution is formed in a culture region in which the cell is retained in the culture tank.

In addition, a cell culture method according to one aspect of the present disclosure, in a cell culture device including a culture tank of culturing a cell in a culture solution, the culture tank being in a shape in which a horizontal sectional area upwardly increases, in which the culture solution is intermittently supplied to the culture tank from a supply unit of the cell culture device, according to the control of a control unit of the cell culture device, and a circulation upward flow is formed in the culture solution, and thus, a plug flow of the culture solution is formed in a culture region in which the cell is retained in the culture tank.

According to the cell culture device and the cell culture method described above, the circulation upward flow is formed in the culture solution in the culture tank, and thus, the plug flow of the culture solution is formed in the culture region where the cell is retained in the culture tank. Here, the plug flow of the culture solution is formed in the culture region, while intermittently supplying the culture solution, according to the control of the control unit, and thus, even in a case where the cell has grown, it is possible to control a supply amount per unit time of the culture solution according to the adjustment of a time zone for supplying the culture solution, or the like. In addition, the culture solution is intermittently supplied to the culture tank, and thus, it is possible to prevent a drift from being generated in the culture tank. Therefore, it is possible to control the supply amount of the culture solution according to the growth of the cell while stabilizing the flow of the culture solution in the culture tank, compared to the case of controlling the supply amount per unit time while consecutively supplying the culture solution.

Here, the supply unit is capable of including a culture solution supply line for supplying the culture solution to the culture tank, and a pump provided on the culture solution supply line, and the control can be performed by the control unit such that a time for driving the pump and a time for stopping the driving of the pump are alternately repeated, and thus, the culture solution can be intermittently supplied to the culture tank.

Thus, in a case where the control is performed by the control unit such that the time for driving the pump and the time for stopping the driving of the pump are alternately repeated, and thus, the culture solution is intermittently supplied to the culture tank, it is possible to realize the intermittent supply of the culture solution with respect to the culture tank, with a simpler configuration.

In addition, the cell culture device is capable of further including a cell distribution detection unit detecting a distribution of the cell in the culture tank, and the control unit is capable of controlling the supply amount per unit time of the culture solution to be supplied to the culture tank by the supply unit, on the basis of the distribution of the cell in the culture tank, detected by the cell distribution detection unit.

As described above, in a case where the supply amount per unit time of the culture solution to be supplied to the culture tank by the supply unit, is controlled on the basis of the distribution of the cell in the culture tank, detected by the cell distribution detection unit, and thus, the distribution of the cell in the culture tank is changed from the assumed situation, it is possible to automatically control the supply amount per unit time of the culture solution in order to correct the change. Therefore, it is possible to more preferably controlling the supply amount of the culture solution according to the growth of the cell, while stabilizing the flow of the culture solution in the culture tank.

In addition, the cell culture device is capable of further including: a culture environment detection unit detecting a culture environment in the culture tank; an adjustment unit adjusting the culture solution to be supplied to the culture tank, in front of the supply unit; and an adjustment control unit controlling the adjustment of the culture solution of the adjustment unit, on the basis of the culture environment in the culture tank, detected by the culture environment detection unit.

As described above, the adjustment in the adjustment unit adjusting the culture solution to be supplied to the culture tank, is controlled on the basis of the culture environment in the culture tank, detected by the culture environment detection unit, and thus, it is possible to automatically control the adjustment for improving the culture environment in the culture tank. Therefore, it is possible to improve the culture environment, while preferably controlling the supply amount of the culture solution according to the growth of the cell in the culture tank.

Hereinafter, a mode for carrying out the present disclosure will be described in detail, with reference to the attached drawings. Furthermore, the same reference numerals will be applied to the same constituents in the description of the drawings, and the repeated description will be omitted.

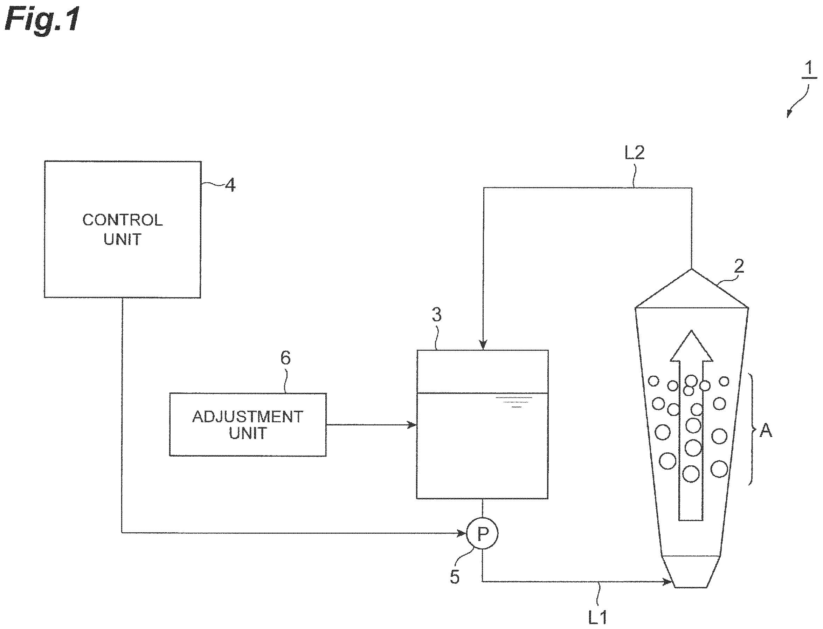

FIG. 1 is a diagram illustrating a schematic configuration of a cell culture device according to one aspect of the present disclosure. A cell culture device 1 includes a culture tank 2 that is a vessel for culturing a cell by a culture solution (a cell culture solution), an adjustment tank 3 of reserving the culture solution to be supplied to the culture tank 2 and of adjusting the culture solution, a control unit 4 performing control relevant to the cell culture device 1, a pump 5 circulating the culture solution in the device by supplying the culture solution into the culture tank 2, and an adjustment unit 6 performing an operation relevant to the adjustment of the culture solution in the adjustment tank 3. In addition, a culture solution supply pipe L1 (the culture solution supply line) supplying the culture solution to the culture tank 2 from the adjustment tank 3, and a culture solution returning pipe L2 (a culture solution returning line) returning the culture solution to the adjustment tank 3 from the culture tank 2, are provided between the culture tank 2 and the adjustment tank 3. The culture solution supply pipe L1 and the pump 5 function as the supply unit supplying the culture solution to the culture tank 2. In addition, the control unit 4 controls the supply of the culture solution with respect to the culture tank 2 according to the supply unit.

A cell that is a culture target of the cell culture device 1 according to this embodiment, is not particularly limited, and for example, it is possible to culture a stem cell in an animal, or the like. The culture solution that is a medium in the cell culture, is suitably selected according to the type of cell that is the culture target.

Furthermore, in the following embodiment, a case where the cell culture device 1 is a general installation type cell culture device, will be described, but the cell culture device 1 may be downsized as a so-called transport device for transporting the culture tank 2. In the case of the downsized cell culture device, there is a case where each unit configuring the device, such as the culture tank 2 and the adjustment tank 3, is downsized or simplified, but the basic configuration is the same.

The culture tank 2 has a shape in which a sectional area in a horizontal direction upwardly increases. As such a shape, for example, the culture tank 2 can be in an inverted conical shape, as illustrated in FIG. 1 or the like, but the shape of the culture tank 2 is not limited to the inverted conical shape. The inside of the culture tank 2 is filled with the culture solution. In addition, in the culture tank 2, a culture region A is formed in which a cell or a carrier to which the cell is attached float in a predetermined height range. Hereinafter, the cell or the carrier to which the cell is attached, will be referred to as a culture cell.

The content of the culture tank 2 is not particularly limited, but can be approximately 10 mL to 10,000 mL. The content of the culture tank 2 can be suitably selected on the basis of the amount of cell to be cultured in one culture tank 2, the size of a cell mass, or the like. In addition, the content of the culture tank 2 can be selected according to handling properties of the culture tank 2, or the like. For example, in a case where the content of the culture tank 2 is approximately 50 mL to 500 mL, the handling properties as the single culture tank 2, are improved.

The culture solution supply pipe L1 is connected to a lower end of the culture tank 2. In addition, the culture solution returning pipe L2 is connected to an upper end of the culture tank 2. The culture solution supply pipe L1 and the culture solution returning pipe L2 connect the culture tank 2 and the adjustment tank 3 together.

The adjustment tank 3 has a function of reserving the culture solution. In addition, the adjustment unit 6 is provided in the adjustment tank 3, and thus, various adjustments can be performed such that the culture solution suitable for the cell culture is obtained. There is a case where a gas concentration in the culture solution decreases according to the culture of the cell. In this case, for example, a gas supply unit such as a microbubble pump, is provided as the adjustment unit 6, and thus, it is possible to adjust the gas concentration in the culture solution. In addition, there is a case where a ratio of a specific component in the culture solution is reduced according to the culture of the cell. In this case, for example, a means supplying the component is provided as the adjustment unit 6, and thus, it is possible to adjust various component ratios in the culture solution. Thus, a means necessary for adjusting the culture solution necessary for the culture of the cell, can be suitably selected, and can be provided, as the adjustment unit 6. Furthermore, gas supply can be performed by only surface aeration or the like. In addition, the adjustment unit 6 may not be provided with respect to the adjustment tank 3, and may be provided in front of the supply unit supplying the culture solution to the culture tank 2.

The control unit 4 has a function of performing various controls relevant to the cell culture device 1. In particular, in the cell culture device 1 according to this embodiment, the control unit 4 has a function of controlling the driving of the pump 5 that is provided on the culture solution supply pipe L1. The details will be described below, but the driving of the pump 5 is controlled by the control unit 4, and thus, it is possible to preferably control a flow rate of the culture solution to be moved in the culture tank 2. In addition, the control unit 4 may also control the adjustment of the culture solution in the adjustment unit 6.

The control unit 4 is configured as a computer provided with hardware such as a central processing unit (CPU), a random access memory (RAM) and a read only memory (ROM) that are a main storage device, a communication module performing communication with respect to other devices, and an auxiliary storage device such as a hard disk. Then, such constituents are operated, and thus, such constituents are operated, and thus, each function as the control unit 4 described below, is exhibited.

The pump 5 that is a control target of the control unit 4, is provided on the culture solution supply pipe L1 connecting the adjustment tank 3 and the lower end of the culture tank 2 together. For example, a peristaltic pump can be used as the pump 5, but the type of pump is not particularly limited.

Furthermore, a drainage line for discharging the culture solution to the outside, a culture solution introduction line for supplying a new culture solution, and the like may be separately provided in the cell culture device 1. In addition, in order to suitably maintain the culture solution in the cell culture device 1 at a temperature according to the culture environment, a part of the cell culture device 1 may be provided in an isothermal device such as an incubator.

A cell culture method of the cell culture device 1 described above, will be described. When the culture of the cell is started by the cell culture device 1, first, a new culture solution is introduced to the adjustment tank 3 from the outside. Then, gas is supplied by the adjustment unit 6, and thus, the culture solution in the adjustment tank 3 is adjusted to a condition suitable for the cell culture. After that, the culture solution is introduced into the culture tank 2 from the lower end of the culture tank 2 through the culture solution supply pipe L1, according to the driving of the pump 5.

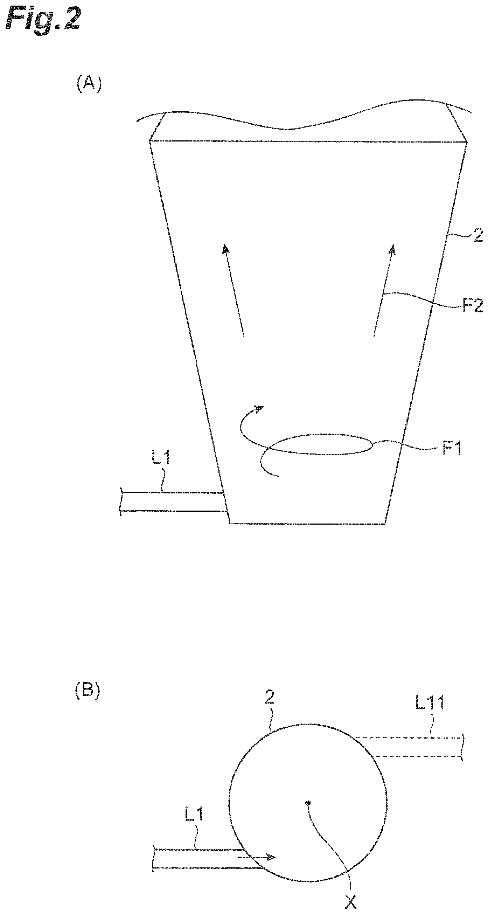

In the culture tank 2, the upward flow of the culture solution is formed. The upward flow will be described with reference to FIG. 2. FIG 2(A) is a diagram illustrating the flow of the culture solution, which is generated in the culture tank 2, and FIG. 2(B) is a sectional view of the culture tank 2 in the horizontal direction, and is a diagram illustrating an attachment position of the culture solution supply pipe L1 with respect to the culture tank 2.

As illustrated in FIG 2(B), the culture solution supply pipe L1 is attached to a position not intersecting with a central axis X extending in an up-down direction in the culture tank 2. That is, the central axis X and the culture solution supply pipe L1 are twisted with each other. In such a state, in a case where the culture solution is supplied from the culture solution supply pipe L1 into the culture tank 2, the culture solution is moved up while being rotated along an inner wall of the culture tank 2 by a turning force generated due to friction between the culture solution and a wall surface in the culture tank 2. That is, as illustrated in FIG 2(A), a turning upward flow F1 that is one type of circulation upward flow, is generated from the lower side to the upper side of the culture tank 2. In this embodiment, the circulation upward flow indicates that a liquid in a tank is moved up while being circulated along an inner wall of the tank.

The turning force due to the friction with respect to the wall surface of the culture tank 2 decreases, as the turning upward flow F1 of the culture solution in the culture tank 2 is moved up, and thus, as illustrated in FIG. 2(A), a upward flow F2 is formed in which the moving-up is more mainly performed than the turning. Accordingly, in the vicinity of the culture region A of the culture tank 2 (refer to FIG. 1), a flow (a plug flow) having an approximately constant flow rate distribution in a moving-up direction on a horizontal surface, is formed.

Furthermore, a formation method of the plug flow is not limited to the method illustrated in FIG. 2, and can be variously changed. For example, as illustrated in FIG. 2(B), in a case where another culture solution supply pipe L11 is provided to be point-symmetric with respect to the culture solution supply pipe L1, on the basis of the central axis X of the culture tank 2, it is possible to equally form the turning upward flow F1 in the culture tank 2. In addition, a method of inputting a spherical body for forming a turning flow in the culture tank 2, or the like, or a method of forming an upward turning flow by changing the shape of the inner wall of the culture tank 2, or the like, are also considered. Thus, the method of forming the upward turning flow, can be suitably changed, and in a case where the turning upward flow F1 is formed in a lower portion in the culture tank 2, it is possible to form the plug flow of the upward flow F2 on the upper side.

In the culture tank 2, the horizontal sectional area upwardly increases, and thus, a flow rate of the upward flow F2 in the plug flow decreases as being directed towards the upper side of the culture tank 2. In the vicinity of the culture region A of the culture tank 2, a state is formed in which a sedimentation rate of the culture cell and the flow rate of the upward flow F2 are balanced. As a result thereof, the culture cells are respectively retained in a predetermined height position. In addition, a shear stress due to the upward flow F1 acts on the culture cell, and thus, the cell that has excessively grown, is crushed, and it is possible to maintain the entire cell diameter.

In a case where the cell has grown, the cell mass in which the cells are aggregated, also becomes larger. That is, the diameter of the culture cell in the culture tank 2 increases, and thus, the sedimentation rate increases. In this case, the flow rate of the upward flow F2 in the culture tank 2 increases, and thus, it is possible to continuously retain the culture cell that becomes larger, in the vicinity of the culture region A.

The culture solution is supplied from the lower end of the culture tank 2 by the culture solution supply pipe L1, and the culture solution is discharged from the culture solution returning pipe L2 on the upper end of the culture tank 2, and is returned to the adjustment tank 3. The culture solution returned to the adjustment tank 3 is supplied to the culture tank 2 according to the driving of the pump 5, while being adjusted to a condition suitable for the cell culture, by the adjustment unit 6. Thus, in the cell culture device 1, in an environment where the culture solution is circulated between the culture tank 2 and the adjustment tank 3, the cell is cultured in the culture tank 2.

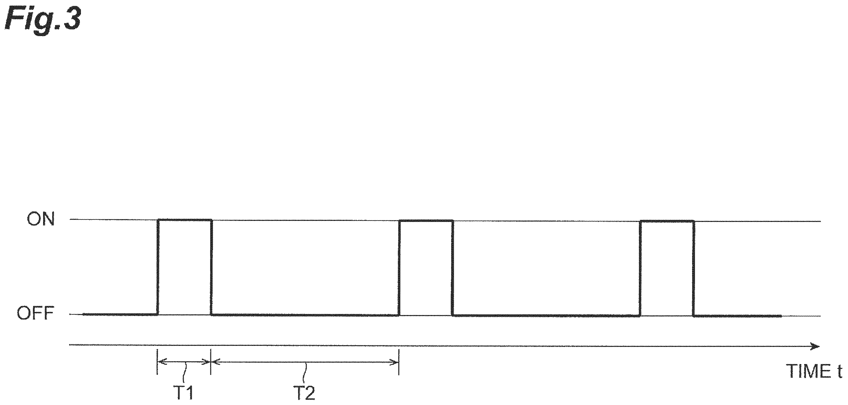

Here, in the cell culture device 1 and the cell culture method according to this embodiment, the culture solution is intermittently supplied to the culture tank 2, and thus, the upward flow F2 that is the plug flow in the culture tank 2, is formed, and the supply amount (the flow rate) of the culture solution per unit time is controlled. Such a point will be described with reference to FIG. 3.

In the cell culture device 1 according to this embodiment, a method of controlling the driving (ON/OFF) of the pump 5 by the control unit 4, is used as one of methods for intermittently supplying the culture solution to the culture tank 2. In FIG. 3, in a case where a horizontal axis is set to a time t, switching (ON/OFF) example of the driving of the pump 5, corresponding to the elapsed time, is illustrated. In the example illustrated in FIG. 3, in a state where the flow rate of the culture solution is set to be constant at the time of driving the pump 5, a driving time T1 for turning on the pump 5 and a stop time T2 for turning off the pump 5, are alternately repeated. The culture solution is supplied to the culture tank 2 during the driving time T1 for turning on the pump 5. On the other hand, the supply of the culture solution with respect to the culture tank 2, is stopped during the stop time T2 for turning off the pump 5. As a result thereof, the amount of culture solution to be supplied to the culture tank 2 during one cycle (T1 + T2), is the amount of culture solution to be supplied according to the driving of the pump 5 during the driving time T1. That is, in a case where the supply amount of the culture solution with respect to the culture tank 2 per unit time according to the driving of the pump 5 is set to X (mL/min), the supply amount of the culture solution with respect to the culture tank 2 per unit time, is X·T1/(T1 + T2) (mL/min), according to the intermittent driving of the pump 5.

Thus, in a case where the culture solution is intermittently supplied to the culture tank 2, it is possible to control the supply amount of the culture solution with respect to the culture tank 2 per unit time, that is, the substantial flow rate of the culture solution to be supplied to the culture tank 2, by changing a ratio between a supply time (the driving time) and a supply stop time (the stop time).

As described above, the culture solution is intermittently supplied to the culture tank 2, and thus, it is possible to reduce a change in the flow rate due to a change in the number of rotations of the pump 5 according to the growth of the cell.

In a case where the cell is cultured in the culture tank 2 while being retained in the culture region A, as with the cell culture device 1, the flow rate is also changed according to the volume, the shape, or the like of the culture tank 2, and the flow rate of the culture solution at the time of sowing a single cell in the culture tank 2 (the supply amount of the culture solution with respect to the culture tank 2 per unit time), for example, is set to approximately 0.2 mL/min. At this time, in a case where the cell mass is upsized according to the growth of the cell according to the culture, for example, there is a case where it is necessary to increase the flow rate of the culture solution, for example, up to approximately 20 mL/min, in order to perform the culture while retaining the cell in the culture region A. That is, there is a case where it is necessary to change the flow rate up to 100-fold speedup, according to the growth of the cell. Thus, there are many cases where the pump 5 capable of greatly changing the flow rate, is expensive, and thus, there is a possibility that device cost increases.

In addition, in a case where the flow rate of the culture solution to be supplied to the culture tank 2, is changed, there is a possibility that the plug flow stabilized in the culture tank 2, is not capable of being formed. For example, in a case where the pump 5 is driven such that the culture solution is introduced into the culture tank 2, at a low flow rate of approximately 0.2 mL/min, as described above, there is a possibility that a drift is generated on the upper side of the culture tank 2, due to the clogging of a nozzle, or the like. In this case, the flow of the culture solution in the culture tank 2 is destabilized, and thus, there is a possibility that the growth of the cell is also affected.

On the other hand, in the cell culture device 1 according to this embodiment, the culture solution is intermittently supplied to the culture tank 2, and thus, it is possible to control the substantial flow rate of the culture solution to be supplied to the culture tank 2. That is, the driving of the pump 5 is set to be constant, and the driving time (a time for turning on the pump 5) and the driving stop time (a time for turning off the pump 5) are adjusted, and thus, it is possible to control the substantial flow rate of the culture solution to be supplied to the culture tank 2. In the case described above, for example, a flow volume according to the driving of the pump 5, is set to 20 mL/min, the driving time T1 of the pump 5 is set to 6 seconds, and the stop time T2 is set to 594 seconds. Then, the substantial flow rate of the culture solution to be supplied to the culture tank 2, is 0.2 mL/min, the flow rate of the culture solution at the time of sowing the single cell in the culture tank 2, can be realized. In addition, in order to change the substantial flow rate of the culture solution to be supplied to the culture tank 2 (the supply amount per unit time), the flow rate according to the pump 5 may not be changed, but a relationship between the driving time T1 and the stop time T2 of the pump 5 may be changed. Thus, in the cell culture device 1 according to this embodiment, even though an expensive pump is not prepared, it is possible to suitably adjust the flow rate of the culture solution according to the growth of the cell.

In addition, as described above, the culture solution is intermittently supplied to the culture tank 2, and thus, the substantial flow rate of the culture solution to be supplied to the culture tank 2 is controlled, and therefore, it is possible to form the plug flow stabilized in the culture tank 2. As described above, in a case where the flow rate of the culture solution is set to a low rate, in a state where the pump 5 is consecutively driven, it is considered that the flow of the culture solution in the culture tank 2 is destabilized, but in a case where the culture solution is intermittently supplied to the culture tank 2, even though a drift is generated on the upper side of the culture tank 2, it is possible to eliminate the drift during a time zone when the supply of the culture solution is stopped. Therefore, it is possible to form the plug flow stabilized in the culture tank 2. In addition, in a case where the stabilized plug flow can be formed, it is possible to extrude an old medium with a new medium, and thus, it is possible to replace the medium while performing the culture in a state where the cell mass floats. Therefore, an operation necessary for a general stirring type culture tank, such as extracting the culture solution, separating the cell from the medium according to centrifugal separation, and suspending in the new medium, is not necessary. In addition, the type of medium is changed, and thus, it is also possible to perform differentiation induction in a state where the cell mass floats.

Further, it is considered that the culture solution is intermittently supplied to the culture tank 2, and thus, the growth of the cell is accelerated, compared to a case where the culture solution is consecutively supplied to the culture tank 2. For example, it is known that in a case where a multipotency stem cell such as an ES cell of a mouse, is not in contact with a carrier or the other cells, the proliferation of the cell does not progress, that is, the growth of the cell is decelerated. On the other hand, as with this embodiment, the culture solution is intermittently supplied to the culture tank 2, and thus, the movement of the carrier and the cell in the culture solution is accelerated. For this reason, a contact opportunity between the carrier and the cell or between the cells, increases, and thus, the growth of the cell (the proliferation) is accelerated.

Furthermore, in the intermittent supply of the culture solution with respect to the culture tank 2, a time for depositing a cell that has excessively grown, and thus, is required to be crushed, in the area of the turning upward flow F1, and the stop time T2 are matched, and thus, a strong shear stress is applied only to the cell that has excessively grown, and thus, can be selectively crushed. Accordingly, the entire cell diameter can be maintained.

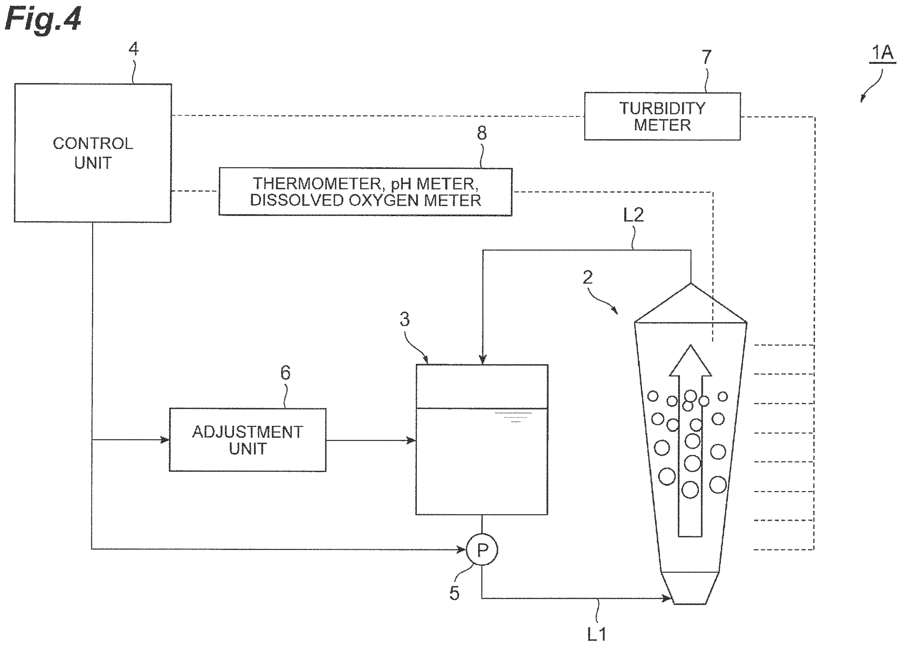

Here, a cell culture device 1A capable of automatically controlling the intermittent supply of the culture solution with respect to the culture tank 2 and the adjustment of the culture solution by the control unit 4, will be described as a modification example of the cell culture device 1 described above. FIG. 4 is a diagram illustrating a schematic configuration of a cell culture device according to a modification example.

The cell culture device 1A includes a turbidity meter 7 that is the cell distribution detection unit for detecting the cell distribution in the culture tank 2, and a thermometer, a pH meter, and a dissolved oxygen meter 8 that are the culture environment detection unit for detecting the culture environment.

The turbidity meter 7 measures the turbidity of the culture solution in the culture tank 2, in a plurality of spots in the up-down direction. In the culture tank 2, the turbidity of the culture solution is changed according to the distribution of the cell. Therefore, the distribution of the turbidity is measured, and thus, it is possible to detect the distribution of the cell in the culture tank 2. Furthermore, a means for detecting the distribution of the cell in the culture tank 2, is not limited to the turbidity meter, and for example, a camera performing optical measurement, or the like may be used. The measurement of the turbidity meter 7 can be performed for a predetermined time (for example, every few hours). Then, a detection result of the turbidity meter 7 is sent to the control unit 4.

The control unit 4 controls the driving of the pump 5, on the basis of information relevant to the distribution of the cell in the culture tank 2, to be detected by the turbidity meter 7. Specifically, for example, as the result of the measurement of the turbidity meter 7, in a case where the distribution of the cell is moved to the lower side from the culture region A, the control unit 4 controls the driving time of the pump 5 such that the driving time is prolonged to increase the supply amount of the medium per unit time with respect to the culture tank 2. In addition, in a case where the distribution of the cell is moved to the upper side from the culture region A, the control unit 4 controls the driving time of the pump 5 such that the driving time is shortened to decrease the supply amount of the medium per unit time with respect to the culture tank 2. In the control unit 4, information in which how to control the driving of the pump 5 is set (for example, a numerical expression or the like) is retained in advance with respect to the information relevant to the distribution of the cell, on the basis of the result of the measurement of the turbidity meter 7, and thus, it is possible to automatically control the intermittent supply of the culture solution with respect to the culture tank 2, on the basis of the distribution of the cell based on the result of the measurement of the turbidity meter 7.

The thermometer, the pH meter, and the dissolved oxygen meter 8 respectively measure the temperature, the pH, and the dissolved oxygen of the culture solution in the culture tank 2. All of the temperature, the pH, and the dissolved oxygen of the culture solution, are requisites affecting the culture of the cell, and the culture environment in the culture tank 2 can be detected by measuring such requisites. Furthermore, the culture environment detection unit for detecting the culture environment, may be at least one of the thermometer, the pH meter, and the dissolved oxygen meter, described above, or may be combined with other measurement devices (for example, an electrical conductivity meter or the like). The measurement of the thermometer, the pH meter, and the dissolved oxygen meter 8, can be performed for a predetermined time (for example, every few hours). Then, detection results of the thermometer, the pH meter, and the dissolved oxygen meter 8 are sent to the control unit 4.

In this case, the control unit 4 controls the adjustment of the culture solution of the adjustment unit 6, on the basis of information relevant to the environment of a culture system in the culture tank 2, to be detected by the thermometer, the pH meter, and the dissolved oxygen meter 8. That is, the control unit 4 functions as the environment adjustment control unit controlling the adjustment of the culture solution to be supplied to the culture tank 2, on the basis of the culture environment in the culture tank 2. Specifically, for example, as the result of the measurement of the dissolved oxygen meter, in a case where the dissolved oxygen in the culture solution is reduced from a predetermined condition, in the adjustment unit 6, the amount of gas to be supplied to the culture solution increases. In addition, heating control is performed with respect to the adjustment tank 3 in which the culture solution is reserved, according to a measurement result of the temperature. In addition, in the adjustment unit 6, the amount of medicinal agent to be added to the culture solution, is changed according to a measurement result of the pH. How to control the adjustment of the culture solution of the adjustment unit 6 is set in advance, on the basis of the measurement result of each or all of the thermometer, the pH meter, and the dissolved oxygen meter, and in the control unit 4, and the control of the adjustment unit 6 is performed as set in advance, corresponding to information obtained from the thermometer, the pH meter, and the dissolved oxygen meter 8, and thus, it is possible to automatically control the adjustment of the culture solution to be supplied to the culture tank 2.

In the cell culture device 1A, it is possible to automatically control the intermittent supply of the culture solution with respect to the culture tank 2, on the basis of the information relevant to the distribution of the cell in the culture tank 2 to be detected by the turbidity meter 7. In addition, it is possible to automatically control the adjustment of the culture solution in the adjustment unit 6, on the basis of the information relevant to the culture environment in the culture tank 2, to be detected by the thermometer, the pH meter, and the dissolved oxygen meter 8. Thus, it is possible to automatically control the intermittent supply of the culture solution to the culture tank 2 or the adjustment of the culture solution in the adjustment unit 6, and thus, it is possible to form a state in which the supply amount of the culture solution is controlled according to the growth of the cell, while stabilizing the flow of the culture solution in the culture tank in a state where the operation of the operator is not required.

Furthermore, in the cell culture device 1A, it has been described that both of the intermittent supply of the culture solution with respect to the culture tank 2 and the adjustment of the culture solution, are automatically controlled, but only one of them may be controlled.

As described above, according to the cell culture devices 1 and 1A and the cell culture method of this embodiment, the circulation upward flow is formed in the culture solution in the culture tank 2, and thus, the plug flow of the culture solution is formed in the culture region A where the cell is retained in the culture tank 2. Here, the plug flow of the culture solution is formed in the culture region A while intermittently supplying the culture solution, according to the control of the control unit 4, and thus, it is possible to control the supply amount per unit time of the culture solution by adjusting the time zone for supplying the culture solution even in a case where the cell has grown. In addition, the culture solution is intermittently supplied to the culture tank 2, and thus, it is possible to prevent a drift from being generated in the culture tank 2, and therefore, it is possible to control the supply amount of the culture solution according to the growth of the cell, while stabilizing the flow of the culture solution in the culture tank 2, compared to the case of controlling the supply amount per unit time while consecutively supplying the culture solution, as with the related art.

In addition, as described in the embodiment, in a case where the control is performed by the control unit 4 such that the time for driving the pump 5 and the time for stopping the driving of the pump 5 are alternately repeated, and thus, the culture solution is intermittently supplied to the culture tank 2, it is possible to realize the intermittent supply of the culture solution with respect to the culture tank 2, with a simpler configuration.

In addition, as with the cell culture device 1A, the supply amount per unit time of the culture solution to be supplied to the culture tank 2, is controlled on the basis of the distribution of the cell in the culture tank 2, detected by the turbidity meter 7 that is the cell distribution detection unit, and thus, in a case where the distribution of the cell in the culture tank 2 is changed from the assumed situation, it is possible to automatically control the supply amount per unit time of the culture solution in order to correct the change. Therefore, it is possible to more preferably control the supply amount of the culture solution according to the growth of the cell, while stabilizing the flow of the culture solution in the culture tank 2.

Further, as with the cell culture device 1A, the adjustment of the culture solution in the adjustment unit 6 is controlled on the basis of the culture environment in the culture tank 2, detected by the thermometer, the pH meter, and the dissolved oxygen meter 8 that are the culture environment detection unit, and thus, it is possible to automatically control the adjustment for improving the culture environment in the culture tank 2. Therefore, it is possible to improve the culture environment while preferably controlling the supply amount of the culture solution according to the growth of the cell in the culture tank 2.

The embodiment described above, represents one example of the present disclosure. The cell culture device and the cell culture method according to the present disclosure, are not limited to the embodiment described above, and may be modified within a range not changing the gist described in each claim, or may be applied to other objects.

For example, in the cell culture device described in the embodiment, the culture solution to be supplied to the culture tank 2 by the culture solution supply pipe L1, is discharged by the culture solution returning pipe L2, and is circulated through the adjustment tank 3. However, it is not necessary that the culture solution is circulated, and the culture solution can be variously changed insofar as the culture solution is supplied into the culture tank 2 from the lower side of the culture tank 2 by the supply unit such as the pump 5, and is discharged from the upper side while forming the plug flow in the culture tank 2.

In addition, in the embodiment described above, a case where the intermittent supply of the culture solution with respect to the culture tank 2 is realized by the pump 5 provided on the culture solution supply pipe L1, has been described. However, the configuration of the supply unit intermittently supplying the culture solution to the culture tank 2, is not limited to the configuration described above. For example, the supply unit, the culture solution tank, and an on-off valve in pipe arrangement connected to the culture solution tank, may be provided, and the intermittent supply of the culture solution with respect to the culture tank 2 may be realized by controlling the on-off valve.

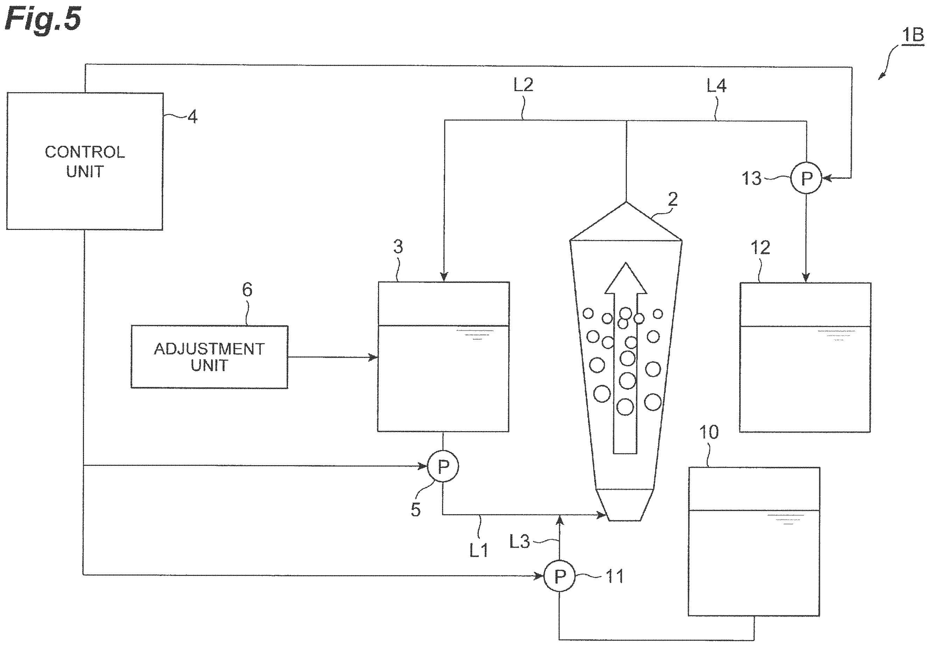

A cell culture device IB capable of performing medium replacement in the plug flow, will be described as a modification example of the cell culture device 1 described above. FIG. 5 is a diagram illustrating a schematic configuration of a cell culture device according to a modification example.

The cell culture device 1B illustrated in FIG. 5 includes a medium reserving tank 10 reserving a new medium, a medium supply pipe L3 for supplying the new medium in the medium reserving tank 10 into the culture tank 2, a medium supply pump 11 controlling the supply of the new medium, a waste solution reserving tank 12 reserving an old medium as a waste solution, a waste solution transfer pipe L4 for removing the old medium from the culture tank 2, and a waste solution pump 13 controlling the removal of the old medium, compared to the cell culture device 1 illustrated in FIG. 1.

In each unit described above, the medium supply pump 11 and the waste solution pump 13 are controlled by the control unit 4, as with the pump 5.

In the cell culture device 1B, the pump 5 is stopped at the time of performing medium replacement according to the plug flow. Then, the medium supply pump 11 and the waste solution pump 13 are controlled by the control unit 4 such that the medium supply pump 11 and the waste solution pump 13 feed a liquid at the same rate. Accordingly, the new medium can be supplied into the culture tank 2 and the old medium can be discarded, while being cultured in the culture tank 2, that is, it is possible to perform the medium replacement in the culture tank 2. An intermittent operation is performed with respect to the medium supply pump 11 and the waste solution pump 13, at the same timing as that of an intermittent operation of the pump 5, or a timing close thereto, and thus, it is possible to form a stabilized plug flow. Therefore, the old medium can be ejected from the tank almost without being mixed with the new medium. Accordingly, it is possible to efficiently perform the medium replacement in the culture tank 2, without any waste.

In addition, it is also possible to change the type of medium at the time of performing the differentiation induction of the cell, or the like, by using the medium replacement method described above. The type of medium can be changed by sterilely replacing the content of the medium reserving tank 10 with another medium. In addition, the type of medium can also be changed by increasing a combination of the medium reserving tank, the medium supply pipe, and a medium feed pump, by connecting a new medium supply pipe to a portion in which the medium supply pipe L3 and the culture solution supply pipe L1 intersect with each other or a portion from the pump 5 to the culture tank 2 in the culture solution supply pipe L1, and by operating the new medium supply pump instead of the medium supply pump 11.

### Industrial Applicability

According to the present disclosure, it is possible to control a supply amount of a culture solution according to the growth of a cell while stabilizing the flow of the culture solution in a culture tank.

### Reference Signs List

1, 1A: cell culture device, 2: culture tank, 3: adjustment tank, 4: control unit, 5: pump, 6: adjustment unit, 7: turbidity meter, 8: thermometer, pH meter, dissolved oxygen meter, A: culture region, L1: culture solution supply pipe, L2: culture solution returning pipe.

## Claims

1. A cell culture device, comprising:
a culture tank of culturing a cell in a culture solution, the culture tank being in a shape in which a horizontal sectional area upwardly increases;
a supply unit supplying the culture solution to the culture tank; and
a control unit controlling the supply of the culture solution of the supply unit,
wherein the culture solution is intermittently supplied to the culture tank from the supply unit, according to the control of the control unit, and a circulation upward flow is formed in the culture solution, and thus, a plug flow of the culture solution is formed in a culture region in which the cell is retained in the culture tank.

2. The cell culture device according to claim 1,
wherein the supply unit includes,
a culture solution supply line for supplying the culture solution to the culture tank, and a pump provided on the culture solution supply line, and
the control is performed by the control unit such that a time for driving the pump and a time for stopping the driving of the pump are alternately repeated, and thus, the culture solution is intermittently supplied to the culture tank.

3. The cell culture device according to claim 1 or 2, further comprising:
a cell distribution detection unit detecting a distribution of the cell in the culture tank,
wherein the control unit controls a supply amount per unit time of the culture solution to be supplied to the culture tank by the supply unit, on the basis of the distribution of the cell in the culture tank, detected by the cell distribution detection unit.

4. The cell culture device according to any one of claims 1 to 3, further comprising:
a culture environment detection unit detecting a culture environment in the culture tank;
an adjustment unit adjusting the culture solution to be supplied to the culture tank, in front of the supply unit; and
an adjustment control unit controlling the adjustment of the culture solution of the adjustment unit, on the basis of the culture environment in the culture tank, detected by the culture environment detection unit.

5. A cell culture method in a cell culture device including a culture tank of culturing a cell in a culture solution, the culture tank being in a shape in which a horizontal sectional area upwardly increases,
wherein the culture solution is intermittently supplied to the culture tank from a supply unit of the cell culture device, according to the control of a control unit of the cell culture device, and a circulation upward flow is formed in the culture solution, and thus, a plug flow of the culture solution is formed in a culture region in which the cell is retained in the culture tank.
